# FASCICULE DE BREVET EUROPEEN

(11) **EP 2 438 114 B1**
(45) Date de publication et mention de la délivrance du brevet: **08.11.2017**
(21) Numéro de dépôt: 10737600.6
(22) Date de dépôt: 04.06.2010
(51) Int. Cl.: C08K 5/3445

(54) **UTILISATION DE MOLECULES PORTEUSES DE GROUPES ASSOCIATIFS COMME DURCISSEURS DE RESINES THERMODURCISSABLES**
VERWENDUNG VON MOLEKÜLEN MIT ASSOZIATIVEN GRUPPEN ALS HÄRTUNGSMITTEL FÜR WÄRMEHÄRTENDE KUNSTHARZE
USE OF MOLECULES HAVING ASSOCIATIVE GROUPS AS HARDENERS FOR THERMOSETTING RESINS

(30) Priorité: 04.06.2009 FR 0953680
(43) Date de publication de la demande: 11.04.2012
(73) Titulaire: Arkema France, 92700 Colombes (FR)
(72) Inventeur: VAN HEMELRYCK, Bruno, 69630 Chaponost (FR); HIDALGO, Manuel, 69530 Brignais (FR)
(86) Numéro de dépôt international: PCT/FR2010/051095
(87) Numéro de publication internationale: WO 2010/139906

(56) Documents cités:
- GB-A- 2 001 065
- US-A- 3 563 957
- US-A- 5 422 042

## Description

La présente invention concerne le domaine des polymères thermodurcissables ou thermodurs utilisés principalement comme matériaux, revêtements ou adhésifs.

L'invention concerne plus particulièrement l'utilisation de molécules spécifiques en tant qu'agents de modification de système durcisseur de polymères thermodurcissables.

Par opposition aux polymères thermoplastiques, lesquels peuvent être transformés et re-transformés à l'aide de la chaleur qui, avec ou sans apport d'énergie mécanique cisaillante, les ramollit et permet leur écoulement, les polymères thermodurcissables constituent des réseaux polymères réticulés chimiquement, c'est-à-dire par le biais de liaisons de réticulation irréversibles de type covalent, qui, une fois obtenus, ne peuvent plus être transformés par l'action de la chaleur. Une résine thermodurcissable, une fois le réseau polymère constitué, devient un réseau polymère thermodur qui ne coulera pas sous l'effet de la chaleur, même avec un apport d'énergie mécanique cisaillante. On a l'habitude de dire qu'un polymère thermodur soumis à l'effet d'une augmentation constante de température, finira par se dégrader avant d'être en mesure de s'écouler, du fait de la solidité du réseau de réticulation constitué par des liaisons covalentes. Il existe de nombreux systèmes pouvant conduire à l'obtention de réseaux réticulés thermodurs, faisant appel à une grande variété de chimies possibles comme par exemple, les réseaux époxy, polyuréthane, phénol-formol, mélamine-formol, silicones , urée-formol, polyester, polyester insaturé. Le chapitre intitulé « Polymer Networks » par Karel Dusek et Miroslava Duskova-Smrckova, dans le volume 3 de la collection Macromolecular Engineering [Wiley-VCH, 2007, eds. K. Matyjaszewski, Y. Gnanou et L. Leibler] cite les principaux systèmes réticulables permettant d'obtenir des réseaux polymères réticulés.

Il est coutumier pour l'homme de l'art de considérer qu'un réseau polymère thermodur est obtenu par le mélange et la réaction conséquente d'au moins deux composants, avec au moins l'un des deux ayant une fonctionnalité supérieure à 2, vis-à-vis de la réaction mise en jeu. Il est également coutumier pour l'homme de l'art, en particulier dans le cas des systèmes de type époxy ou polyuréthane, d'appeler le composant porteur des fonctions époxy ou isocyanate, « la résine », et d'appeler le composant porteur des fonctions amine ou alcool, « le durcisseur ». Un autre type de langage qui peut être rencontré dans ce domaine est celui qui attribue au réseau polymère en formation ou final, également, l'appellation « résine ». Ainsi il n'est pas rare d'entendre parler, par exemple, de résines époxy, polyuréthane ou polyester. Concernant les systèmes époxy, en particulier, il est donc, nécessaire de tenir compte du contexte pour savoir si l'appellation résine époxy s'applique au composé portant les fonctions réactives oxyrannes de départ (ci-après désignée résine époxy de base) ou s'il s'agit du réseau final, après réaction avec le durcisseur.

Le durcisseur, selon cette appellation est, donc, un composé souvent polyfonctionnel portant des motifs amine ou alcool, réactifs. En mélange avec ce durcisseur, il est possible d'inclure, entre autre, des composés inertes vis-à-vis de la réaction (comme des solvants),ou au contraire des solvants ou diluants réactifs permettant de contrôler la réaction et d'ajuster certaines propriétés mécaniques du produit final, ainsi que des catalyseurs permettant d'accélérer la réticulation des composants réactifs.

Le document GB 2 001 065 décrite un composé comportant un groupe 2-imidazolidinone comme agent de réticulation d'une résine époxy. Cependant, l'enseignement de ce document ne suggère pas d'utiliser un tel composé pour promouvoir l'adhérence de la résine époxy à un support.

L'invention a donc pour but l'utilisation d'un type particulier de molécules telles que des amines ou des alcools porteuses de motifs associatifs comme durcisseur de résine thermodurcissable, en combinaison avec des durcisseurs amine ou alcool usuels, pour augmenter l'adhérence à un support de la résine thermodurcissable. Il est ainsi possible de former des matériaux, revêtements ou adhésifs ayant des propriétés améliorées, notamment une meilleure adhérence aux supports, et par exemple, une meilleure résistance chimique, une meilleure flexibilité, un temps ouvert ou un temps de prise optimaux.

Les inventeurs ont montré dans l'exemple 4 que ces molécules spécifiques, en association avec un durcisseur classique, permettent un renforcement de l'adhésivité de la résine époxy finale.

De plus, comme illustré dans les exemples 1 à 3, ces molécules spécifiques, permettent :
- une excellente catalyse de la réaction de polymérisation, de sorte que l'utilisation d'un accélérateur de prise n'est plus nécessaire,
- une flexibilité améliorée de la résine finale.

L'invention a donc pour objet l'utilisation en tant que durcisseur de résine thermodurcissable, d'une molécule porteuse de groupe associatif et, de préférence d'une molécule porteuse de groupe associatif de formule (I) : 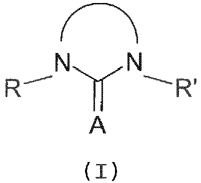 dans laquelle :
R désigne un motif contenant au moins une fonction réactive, de préférence une fonction alcool, thio ou amine,
R' désigne un atome d'hydrogène,
A désigne un atome d'oxygène ou de soufre, de préférence un atome d'oxygène,
en combinaison avec un co-durcisseur portant des fonctions amine ou alcool, pour augmenter l'adhérence à un support de la résine thermodurcissable.

Une telle utilisation présente des avantages considérables par rapport aux durcisseurs usuels, puisqu'elle permet de s'affranchir partiellement ou totalement de l'utilisation d'un accélérateur de prise et/ou d'un agent flexibilisant et/ou d'un promoteur d'adhérence de la résine finale.

Des exemples d'utilisation des différents agents de modification d'une résine thermodure sont détaillés par exemple dans l'ouvrage de Petrie Edward M., Epoxy adhesives Formulations, Chemical Engineering.

Ainsi, il est coutumier pour l'homme de l'art des résines époxy de recourir à une formulation qui puisse nécessiter l'incorporation à la fois d'un catalyseur, d'un agent flexibilisant et d'un agent d'adhérence sous forme de produits distincts.

Un catalyseur pour résines époxy est souvent une amine tertiaire incorporée au durcisseur. La plupart des catalyseurs ne sont cependant pas réputés pour promouvoir la flexibilité dans la résine finale.

Un agent flexibilisant peut être soit introduit au niveau de la résine époxy de base porteuse des fonctions oxirannes, soit au sein du durcisseur. Il s'agit d'une résine plus flexible que la résine époxy de base ou d'un réticulant dont la structure moléculaire est plus mobile que celle du durcisseur, et dans ce cas il s'agit alors d'un co-réticulant. Mais dans les deux cas, l'agent modifiant possède des masses moléculaires relativement hautes qui ont pour effet d'accroître la distance entre les noeuds de réticulation par son incorporation dans le réseau polymère final, avec une diminution importante des propriétés mécaniques de la résine finale. Une variante de ce mode de modification d'une des composantes principales d'un époxy et qui vise à flexibiliser la résine finie est l'hybridation préalable. Il s'agit d'une modification au stade de l'obtention de la résine époxy de base qui permet de produire des résines époxy de base modifiées, telles que époxy-polyamide, époxy-vinyl ou époxy-polysulfure. Certains de ces modifiants hybrides améliorent l'adhérence (cas des epoxies obtenus entre une résine époxy de base telle qu'une résine liquide de type diglycydiléther du bis-phénol A ou « DGEBA » et un polyamide), mais au détriment des propriétés thermomécaniques. Selon ce principe d'hybridation préalable des réactants d'un thermodur bi-composant, d'autres flexibilisants oligomères ou polymères peuvent être utilisés, dans la résine de base ou bien dans le durcisseur, avec par exemple des prépolymères alkyle diisocyanates réagis avec les fonctions alcools d'une résine époxy de base, ou bien encore avec un polymère silicone à terminaisons amines qui est alors employé en co-réactant du durcisseur polyamine. D'autres additifs ou co-réactants polymères ont été développés pour permettre plus de flexibilité de la résine finale, comme des dérivés liquides de polybutadiène permettant l'introduction d'une phase élastique dans le thermodur. Enfin les polysulfures peuvent être utilisés soit en agent flexibilisants, soit comme durcisseurs dans un ratio courant 1:1 avec la résine époxy de base mais dans ce cas ils doivent être absolument catalysés par une amine tertiaire telle que DMP-30 par exemple. De plus leur odeur peut nécessiter l'ajout d'additifs spécifiques.

Outre leur coût élevé, tous ces polymères ou prépolymères, modifiés en vue de pouvoir permettre une flexibilité plus importante de la résine finale, présentent en commun la nécessité de modifier profondément la mise en oeuvre du thermodur par rapport à sa recette originelle lorsqu'un besoin de flexibilité s'impose en application. Ainsi il est parfois nécessaire d'introduire un nouveau type d'additif dans la recette époxy tel qu'un agent compatibilisant ou agent de couplage permettant de limiter les problèmes de déphasage et d'hétérogénité à l'intérieure de la résine flexibilisée, ou bien encore il est nécessaire d'introduire un additif réduisant la viscosité supplémentaire causée par le recours à une composante polymère thermoplastique (cas des époxy-nylon par exemple).

Une autre catégorie d'agents promoteurs de flexibilité est celle des diluants, réactifs ou non. Les diluants non réactifs sont formellement des plastifiants et non des flexibilisants, ils entraînent des dégradations des propriétés mécaniques des résines époxy, et parfois un phénomène d'exudation, particulièrement indésirable pour les adhésifs.

Les diluants réactifs sont non migrants car ils participent à la construction du réseau polymère. Il s'agit souvent de molécules à longue chaîne, à fonctionnalité monoglycidyle, mais des diluants réactifs à fonctionalité di-glycidyle peuvent contribuer à la densité du réseau thermodur final, sans toutefois améliorer l'adhérence. Ils sont traditionnellement associés à la composante époxy de base de la résine du fait de leur compatibilité.

Parmi les agents promoteurs d'adhérence des thermodurs, les organosilanes constituent une famille largement utilisée, bons promoteurs d'adhérence entre la résine époxy réticulée et un support minéral porteur de fonctions hydroxyles en surface, par exemple une charge minérale (comme une argile, des alumines), une fibre de verre de renforcement de composite, une céramique. Les organosilanes sont utilisés également pour accroître l'adhérence de l'époxy sur un support métallique. Ils peuvent être employés en revêtement primaire directement sur le support ou bien être incorporés à la formulation du thermodur. Cependant l'utilisation en adhérence sur métaux étant optimale lorsque l'organosilane est appliqué en couche primaire sur le support métallique, cela implique dans ce cas une étape de pré-traitement avant le dépôt du thermodur. Les organosilanes présentent toutefois une sensibilité à l'humidité qui peut rendre leur usage délicat et leur efficacité aléatoire. De plus leur usage sur support polymère nécessite que ce dernier possède à sa surface des groupes réactifs libres capables de réagir avec les silanes, typiquement des groupements OH, si bien que les applications faisant appel aux organosilanes concernent surtout la réalisation de composites dont on veut accroître les propriétés de résistance mécanique, et la durabilité par un couplage entre le liant organique thermodur et le renfort minéral ou métallique.

Une autre sorte d'agent favorisant l'adhérence de la matrice organique polymère époxy sur support minéral est celle des complexes organo-métalliques (titanate ou zirconate par exemple). Ces complexes sont toutefois délicats à appliquer en raison du haut risque de surdosage, leur efficacité optimale étant obtenue pour la quantité assurant la formation d'une mono-couche en surface du support. Leur usage est donc plutôt réservé au traitement de surface de charges minérales, afin de faciliter leur dispersion dans la matrice organique. Il s'agit alors d'une fonction d'agent de couplage entre charge minérale et résine, qui n'apporte pas d'amélioration de l'adhérence entre résine thermodure et un support minéral ou métallique. D'autres complexes organo-métalliques à base de chrome ou de cobalt ont été étudiés mais d'usage limité, en raison de leur toxicité.

Selon l'invention, l'utilisation en tant que durcisseur de résine thermodurcissable, d'une molécule porteuse de groupe associatif telle que définie ci-dessus permet ainsi d'accélérer la prise d'une résine thermodure bi-composant, d'apporter de la flexibilité sans pour autant nuire gravement aux propriétés mécaniques de la résine finale. L'utilisation selon l'invention permet aussi de renforcer l'adhérence de la résine sur des supports non seulement minéraux ou métalliques, mais également polymères non porteurs de fonctions hydroxyles tel le PMMA par exemple. Cette dernière situation d'adhérence entre résine thermodure et polymère est illustrée par la possibilité d'obtenir selon l'invention une adhérence entre polymères de nature différente comme dans le cas par exemple du dépôt d'une couche de thermodur de type polyuréthane sur une sous-couche thermodur époxy.

Dans un mode de réalisation préféré de l'invention, la molécule porteuse de groupe associatif comprenant un hétérocycle azoté est utilisée comme durcisseur d'au moins deux résines thermodurcissables, de préférence différentes.

Ce mode de réalisation est particulièrement avantageux puisqu'il permet d'augmenter l'adhérence entre deux résines en s'affranchissant de l'utilisation d'une colle qui présente l'inconvénient possible d'une évaporation de solvant.

Ce mode de réalisation permet aussi de s'affranchir de l'utilisation d'une résine thermodurcissable partiellement modifiée par un réactif, résine mise à réagir ensuite avec une autre résine. Cette seconde étape de mise en oeuvre peut en effet être difficile si elle nécessite une activation par chauffage, ou bien poser des problèmes pour l'environnement si la réaction doit se faire à température ambiante mais en mettant en oeuvre une chimie de groupes très réactifs comme les isocyanates pouvant nuire à la santé sur le lieu de mise en oeuvre.

A titre d'exemple de la présente invention et en ce qui concerne plus particulièrement les résines thermodurcissables à fonctions époxy, les défauts endémiques de ces époxy que sont l'excès de rigidité et leur manque d'adhérence, sont pour la première fois améliorés par un même agent de modification, sous forme de molécule porteuse de groupe associatif. Jusqu'à présent la modification de la souplesse de la résine Epoxy finie était obtenue soit par modification de la pré-résine ou résine de base porteuse des fonctions époxydes, soit par adjonction de solvant réactif tel qu'un mono-époxyde gras. L'adhérence de la résine Epoxy finie était, de son côté, améliorée par l'adjonction d'agents promoteurs d'adhérence de type sylilé par exemple. Si bien que les deux propriétés, rigidité et adhérence faisaient intervenir des modifications importantes du réseau polymère et une complication de sa mise en oeuvre en termes de formulation.

Par durcisseur, on entend au sens de la présente invention, un composé capable de provoquer une réticulation chimique d'un réseau polymère, par le biais de liaisons de réticulation irréversibles de type covalent, qui, une fois obtenus, ne peuvent plus être transformés par l'action de la chaleur.

Les motifs associatifs selon l'invention, sont des motifs comprenant des hétérocycles azotés, capables de créer entre motifs d'au moins deux molécules différentes, des liaisons physiques complémentaires de type liaison hydrogène.
Par "groupes associatifs", on entend des groupes susceptibles de s'associer les uns aux autres par des liaisons hydrogène, ioniques et/ou hydrophobes. Il s'agit selon un mode préféré de l'invention de groupes susceptibles de s'associer par des liaisons hydrogènes, comprenant un hétérocycle azoté, de préférence diazoté, généralement à 5 ou 6 chaînons, et comprenant de préférence un groupe carbonyle. Des exemples de groupes associatifs utilisables selon ce mode préféré de l'invention sont les groupes imidazolidinyle, bis-uréyle, uréido-pyrimidyle. Le groupe imidazolidinyle est préféré.

Selon l'invention, ces molécules porteuses de groupes associatifs sont utilisées en combinaison avec des durcisseurs usuels de type amine ou alcool, ce qui permet d'ajuster les propriétés des résines thermodures obtenues telles que l'adhérence aux supports et la flexibilité.

Ainsi, selon l'invention l'utilisation d'une molécule porteuse de groupe associatif comprenant un hétérocycle azoté a lieu en combinaison avec un co-durcisseur, c'est-à-dire un durcisseur usuel, pour augmenter l'adhérence de ladite résine à un support..

Comme cela est présenté dans l'exemple 4, l'utilisation d'une telle molécule porteuse de groupe associatif en combinaison avec un co-durcisseur permet d'augmenter l'adhérence à un support d'une résine thermodurcissable par rapport à une résine qui ne comprend pas ce type de molécule porteuse de groupe associatif. De préférence, dans la molécule porteuse de groupe associatif de formule (I) R est choisi parmi H₂N-(CH₂)ₙ-, HS-(CH₂)ₙ-, HO-(CH₂)ₙ- Où n représente un entier compris entre 1 et 18.

En particulier, les molécules porteuses de groupements associatifs comprenant en plus d'une hétérocycle azoté, au moins une fonction amine ou alcool sont particulièrement préférées. De plus, lorsque ces molécules présentent un caractère hydrosoluble, ce qui est le cas notamment lorsque dans les formules ci-dessus n est égal à 1 ou 2 dans les motifs H₂N-(CH₂)ₙ-, HS-(CH₂)ₙ-, HO-(CH₂)ₙ-, cela permet de les utiliser comme durcisseur de résines thermodurcissables dans l'eau ou dans une émulsion aqueuse. Ce mode de réalisation présente un grand intérêt industriel et commercial pour l'obtention, en particulier, de revêtements et d'adhésifs sur base aqueuse.

L'invention a donc aussi pour objet l'utilisation d'une molécule de formule (I), caractérisée en ce que n est égal à 1 ou 2, et en ce que la résine thermodurcissable se trouve dans l'eau ou dans une émulsion aqueuse.

Ainsi, ladite molécule porteuse de groupe associatif est de préférence choisie parmi : la 1-(2-aminoéthyl)imidazolidone, aussi connue comme 1-(2-aminoéthyl)imidazolidine-2-one (UDETA), la 1-(2-hydroxyéthyl)imidazolidone (HEIO), 1-(2-[(2-aminoéthyl)amino]éthyl)imidazolidone (UTETA), la 1-(2-{2-[(2-aminoéthylamino]éthyl}amino) éthyl]imidazolidone (UTEPA), la N-(6-aminohexyl)-N'-(6-méthyl-4-oxo-1,4-dihydropyrimidin-2-yl)urée (UPy).

Les composés UDETA, UTETA, UTEPA peuvent être obtenus par réaction de l'urée avec une polyamine. Par exemple, l'UDETA, l'UTETA et l'UTEPA peuvent respectivement être préparées en faisant réagir de l'urée sur la diéthylène triamine (DETA), la triéthylène tétramine (TETA) et la tétraéthylène pentamine (TEPA). Le composé HEIO peut être obtenu par réaction de l'urée avec le diamine alcool correspondant, à savoir le 2-[(2-aminoéthyl)amino] éthanol.

Comme indiqué ci-dessus, bien que les molécules porteuses de groupements associatifs puissent être utilisées seules comme durcisseur de résine thermodurcissable, ces molécules sont de préférence utilisées en mélange avec d'autres durcisseurs.

Ainsi, les molécules porteuses de groupements associatifs sont utilisées en combinaison avec des durcisseurs usuels à hauteur de 0,1 à 50% en poids par rapport au poids total de la combinaison.

Parmi les composés pouvant être utilisés comme durcisseurs usuels ou co-durcisseurs selon l'invention on peut citer :
- les alkyl- ou aryl- amines et notamment les éthylène polyamines linéaires telles que l'éthylène diamine, la diéthylène triamine (DETA), la triéthylène tétramine (TETA) et la tétraéthylène pentamine (TEPA)), les diamines cycliques notammment le 1,2-diamino cyclohexane, l'isophorone diamine, la N,N'-diisopropyle isophorone diamine, les diamines primaires aromatiques telles que la 4,4'-méthylène dianiline, la méthylene bis 4,4'-orthochloroaniline (ou MBOCA), les isomères de xylène diamine, telles que la diéthyltoluènediamine (ou DETDA)
   - les adduits d'oxyde d'éthylène ou oxyde de propylène avec une polyamine telle que la DETA, par exemple l'hydroxyéthyldiéthylène triamine, les polyétheramines commercialisées par Hunstman sous la dénomination commerciale Jeffamine® D-2000, T-403
   - les adduits DGEBA-amines aliphatiques en excès de fonctions amine relativement aux glycidyles,
   - les polyamidoamines par exemple Versamid® 140 de la société Cognis Corp., Epikure® 3090 de la société Hexion,
   - les polyamides comme Epi-cure® 3090, et Epikure® 3100-ET-60 de la société Hexion,
   - les amidoamines obtenus par condensation entre un acide gras et une polyamine comme Ancamide®-260A®, et Ancamide® 501 d'Air Products
   - les polyamides « flexiblisés » comme Epi-cure® 3164 de la société Hexion,
   - les polymercaptans tels que Capcure® 3830-81, de la société Cognis Corp.,
   - les bases de Mannich obtenus par réaction entre (poly)amine, formaldéhyde et (alkyl)phénols comme Epi-cure® 190, 195, 197 de la société Hexion,
   - les cétimines par exemple Epikure® 3502 de la société Hexion,
   - la dicyandiamide (DICY), AMICURE® CG-1200 d'Air Product,
   - les résines époxy poly-ols de base pouvant réticuler les polyisocyanates par exemple Epikote® 1007 et 1009 de la société Hexion et
   - les polyols et ester-polyols durcisseurs de polyuréthane.

Dans un mode de réalisation de l'invention, la molécule porteuse de groupe associatif selon l'invention peut être utilisée en combinaison avec un agent accélérateur de prise et/ou un agent flexibilisant et/ou un agent promoteur d'adhérence ou même en combinaison avec une autre molécule porteuse de groupement associatif. On peut ainsi envisager par exemple l'utilisation d'UDETA et HEIO comme durcisseur ou co-durcisseur d'une résine polyuréthane-polyurée.

L'accélérateur de prise époxy ou polyuréthane peut être une amine tertiaire telle que les catalyseurs pour polyuréthanes JEFFCAT® de la société Huntsman, un phénol tel l'Epikure® 3253 de la société Huntsman, les acides de Lewis pour catalyser la réaction sur les oxirannes d'époxy ou encore la 2-ethyl-4-methyl imidazole (EMI).

L'agent flexibilisant peut être une résine difonctionnelle plus flexible que la résine de base et être alors employée seule ou en co-réticulation avec le durcisseur usuel ou bien l'agent flexibilisant peut être trouvé parmi des molécules à longue chaîne, à fonctionnalité monoglycidyle, ou parfois diglycidyle (cas des époxy).

L'agent promoteur d'adhérence, peut être choisi parmi un organosilane, un complexe organo-métallique de type titanate ou zirconate, et de manière plus générale choisi parmi les composés cités plus haut.

D'autres additifs peuvent être utilisés en combinaison avec les molécules selon l'invention. Il s'agit par exemple de solvants ou de diluants réactifs ou non, de catalyseurs de la réaction de réticulation, et de composés mono-fonctionnels autres que les molécules porteuses de groupes associatifs selon l'invention. Parmi les solvants non réactifs on peut citer Epodil®748 d'Air Products, parmi les additifs non réactifs on peut citer le dibutyl phtalate, le brai de charbon, l'huile de pin, parmi les diluants réactifs des époxy on peut citer les alkyls éther de glycidyle, l'oxyde de styrène, le diglycidyle éther du butanediol, parmi les catalyseurs de réticulation ont peut citer la dicyandiamide (DICY) (ex. AMICURE CG-1200 d'Air Products), des amines tertiaires telles que Epikure®3253 de Huntsman, toutes les amines tertiaires de la gamme JEFFCAT® de Huntsman, parmi les charges particulièrement appréciées dans le cadre de l'invention on peut citer le talc, la silice calcinée, l'alumine, les silicates, les argiles, le carbonate de calcium, le trioxyde d'aluminium en tant qu'agent retard de feu, les poudres de métaux, les nanotubes de carbones comme agent de conduction thermique ou électrique.

Par résine thermodurcissable, on entend au sens de la présente invention un polymère pouvant être réticulé chimiquement par un durcisseur, en une résine thermodure, qui, une fois obtenue, ne peut plus être transformée par l'action de la chaleur. En d'autres termes, une résine thermodurcissable, une fois le réseau polymère constitué, devient un réseau polymère thermodur qui ne coulera pas sous l'effet de la chaleur, même avec un apport d'énergie mécanique cisaillante.

Parmi les résines thermodurcissables on préfère celles comprenant des motifs époxy, isocyanate ou acide, tels que ceux qui conduisent à l'obtention de réseaux thermodurs de type époxy, polyuréthane, ou polyester, par réaction avec une molécule porteuse de groupes associatifs portant, en plus d'un hétérocycle azoté, une fonction amine ou alcool.

S'agissant des résines époxy à réticuler à l'aide du durcisseur selon l'invention, a titre d'exemple on peut citer des résines époxydées présentant une fonctionnalité, définie comme le nombre de fonctions époxydes par molécule, au moins égale à 2, telles que l'éther diglycidylique du bisphénol A, le diépoxyde de butadiène, le 3,4-époxycyclohexane-carboxylate de 3,4-époxycyclohexylméthyle, le dioxyde de vinylcyclohexène, le 4,4' -di (1,2-époxyéthyl) diphényléther, le 4,4' (1, 2-époxyéthyl) -biphényle, le 2,2-bis (3 ,4-époxycyclohexyl) - propane, l'éther diglycidylique du résorcinol, l'éther diglycidylique du phloroglucinol, le bis(2,3-époxycyclo pentyl) éther, le 2-(3,4-époxy) cyclohexane-5, 5-spiro(3,4- époxy)cyclohexane-m-dioxane, l'adipate de bis-(3,4-époxy-6méthyl-cyclohexyle), le N,N-m-(phénylènebis-4,5-époxy-1,2-cyclohexane-dicarboxamide), un composé diépoxy contenant un cycle hydantoïne, etc. De telles résines peuvent généralement être représentées par la formule (II) : 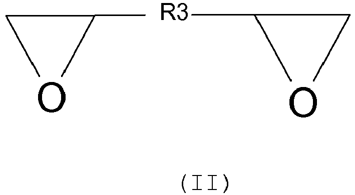 dans laquelle R3 est un groupement de formule -CH2-O-R4-O-CH2- dans laquelle R4 est un groupement divalent choisi parmi les groupements alkylène ayant de 2 à 12 atomes de carbone et ceux comprenant au moins un cycle aliphatique ou aromatique substitué ou non.On peut aussi utiliser des résines polyépoxydées comportant trois ou plus groupes époxydes par molécule comme, par exemple, l'éther triglycidique du p-aminophénol, les éthers polyarylglycidiques, le 1,3,5-tri(1,2-époxy)benzène, la 2,2',4,4'-tétraglycidoxybenzophénone, le tétraglycidoxytétraphényléthane, l'éther polyglycidylique de la résine phénolformaldéhyde de type novolaque, l'éther triglycidylique du glycérol, l'éther triglycidylique du triméthylolpropane et le tétraglycidyl 4,4'-diaminodiphénylméthane.

S'agissant des résines isocyanates à réticuler selon l'invention, on peut citer l'hexaméthylènedisiocyanate (HMDI), les triméthylhexaméthylènediisocyanates (TMDIs) tels le 2,2,4-triméthylhexaméthylènedisiocyanate et le 2,4,4-triméthylhexaméthylènediisocyanate, les undécanes triisocyanates (UNTIs), le 2-méthylpentane diisocyanate, l'isophorone diisocyanate, le norbornane diisocyanate (NBDI), le 1,3-bis (isocyanatométhyl)cyclohexane (XDI hydrogéné), le 4,4'-bis(isocyanatocyclohexyl)méthane (H12MDI), le 2,4- ou 2,6-toluène diisocyanate (TDI), les diphénylméthane diisocyanates (MDIs),le 1,5-naphtalène diisocynanate (NDI), le p-phénylène diisocyanate (PPDI, les adduits comprenant au moins deux fonctions isocyanates et formés par condensation entre composés comportant au moins deux fonctions isocyanates parmi ceux cités et des composés porteurs d'autres fonctions réactives avec les fonctions isocyanate, comme par exemple des fonctions hydroxyle, thiol ou amine.

Parmi les polyisocyanates peuvent être cités les polyisocyanates modifiés, tels que ceux contenant des groupements carbodiimides, des groupements uréthanes, des groupements isocyanurates, des groupements urée ou des groupements biurée.

S'agissant des polyols permettant de réticuler les polyisocyanates pour l'obtention de polyuréthanes et auquels peut être incorporé directement la molécule porteuse de fonction associative selon l'invention telle que le HEIO, on peut citer notamment le glycérol, l'éthylène glycol, le triméthylolpropane, le pentaérythritol, les polyétherpolyols, par exemple ceux obtenus par condensation d'un oxyde d'alkylène ou d'un mélange d'oxydes d'alkylène avec le glycérol, l'éthylène glycol, le triméthylolpropane, le pentaérythritol, les polyesterspolyols, par exemple ceux obtenus d'acides polycarboxyliques, notamment l'acide oxalique, l'acide malonique, l'acide succinique, l'acide adipique, l'acide maléïque, l'acide fumarique, l'acide isophtalique, l'acide téréphtalique, avec le glycérol, l'éthylène glycol, le triméthylolpropane, le pentaérythritol.

Les polyétherpolyols obtenus par addition d'oxydes d'alkylènes, en particulier l'oxyde d'éthylène et/ou l'oxyde de propylène, sur les amines aromatiques en particulier le mélange de 2,4 et 2,6 de toluène diamine conviennent également.

Comme autres types de polyols, on peut citer notamment les polythioéthers à terminaison hydroxyle, les polyamides, les polyesteramides, les polycarbonates, les polyacétals, les polyoléfines et les polysiloxannes.

S'agissant des résines polyester et polyester insaturés obtenus par réaction d'un polyacide avec un polyol on peut citer pour la composante acide l'acide succinique, l'acide pentanedioïque, l'acide adipique, l'acide maleique, l'acide fumarique, l'acide itaconique ainsi que les anhydrides de ces acides, l'acide heptanedioïque, l'acide octanedioïque, l'acide azélaïque, l'acide sébacique, l'acide undécanedioïque, l'acide dodécanedioïque, l'acide brassylique, l'acide tetradécanedioïque, l'acide hexadécanedioïque, l'acide octadécanedioïque, l'acide octadécènedioïque, l'acide eicosanedioïque, l'acide docosanedioïque et les dimères d'acides gras contenant 36 cabones.

Les dimères d'acides gras mentionnés ci-dessus sont des acides gras dimérisés obtenus par oligomérisation ou polymérisation d'acides gras monobasiques insaturés à longue chaîne hydrocarbonée (tels que l'acide linoléïque et l'acide oléïque), comme décrit notamment dans le document EP 0 471 566.

Lorsque le diacide est cycloaliphatique, il peut comporter les squelettes carbonés suivants : norbornyl méthane, cyclohexylméthane, dicyclohexylméthane, dicyclohexylpropane, di(méthylcyclohexyl), di(methylcyclohexyl)propane.

Lorsque le diacide est aromatique, il est choisi parmi l'acide phtalique, téréphtalique, isophtalique, tétrahydrophtalique, trimellitique et les diacides naphtaléniques, ainsi que les anhydrides de ces acides.

S'agissant des polyols, composé dont la molécule comporte au moins deux groupes hydroxyle,permettant de réticuler les polyacides pour l'obtention de polyesters on peut citer les éthylèneglycol, propylèneglycol, butylèneglycol, le 1,6-hexaméthylène glycol, diéthylèneglycol, dipropylèneglycol, néopentylglycol, triéthylèneglycol, glycérol, triméthyloléthane, triméthylolpropane, pentaérythritol, le 1,3 triméthylène glycol, le 1,4-tetraméthylèneglycol, le 1,8 octaméthylèneglycol, le 1,10-décaméthylène glycol, le 1,4-cyclohexane diméthanol, polyetherdiols tels que le PEG, le PPG ou le PTMG, des motifs diacide carboxylique tels que l'acide terephtalique et des motifs glycol (ethane diol) ou butane diol.

On peut également obtenir des polyesters-amides lorsqu'un mélange ternaire d'HEIO, d'UDETA et de polyol est mis en oeuvre avec un des diacides cités.

L'invention sera mieux comprise à la lumière des exemples non limitatifs suivants.

### EXEMPLES

### Protocole de préparation des résines époxy finies pour mises en oeuvre dans les exemples 1 à 4.

Les formulations de résines époxy selon l'invention ont été obtenues de la façon suivante :
- pesée de 100g ou 25g d'une résine époxy de base dans un bécher en plastique jetable, puis
- ajout de la quantité nécessaire de durcisseur calculée selon le principe stoechiométrique connu de l'homme de l'art, c'est à dire un équivalent NH par equivalent époxyde. La masse en grammes de durcisseur à ajouter est ainsi égale à (HEW/EGC) x masse de résine de base engagée, en grammes, avec HEW (equivalent en poids d'hydrogène ou « Hydrogen Equivalent Weight » en anglais exprimé en g/eq) défini par la masse molaire du durcisseur en grammes divisée par le nombre d'hydrogènes actifs, le nombre de NH en l'occurrence, chaque NH étant capable de réagir avec un groupement époxyde, et EGC (Contenu en groupes epoxy ou « Epoxy Group Content » en anglais exprimé en millimole de fonctions époxyde par kilogramme de résine époxy de base), EGC et HEW étant indiquées par les fournisseurs de résine de base et de durcisseur respectivement,

- ajout, simultanément au durcisseur ou par l'intermédiaire du durcisseur après homogénéisation rapide à l'aide d'un bâtonnet, de la molécule porteuse de la fonction associative selon l'invention, à hauteur de quelques % poids de la résine finale, et
- mélangeage avec un agitateur mécanique pendant 1 minute.

### Exemple 1 (illustratif) : Accélération de la prise d'une résine époxy

- réalisation des formulations selon le protocole commun décrit ci-dessus.
- suivi de la température en fonction de l'avancement de la réticulation exothermique à l'aide d'un thermocouple plongeant. L'effet recherché de l'ajout d'un catalyseur est de raccourcir le temps de prise d'un mélange résine-durcisseur jugé trop lent. Le temps ouvert défini par le temps où il est possible d'utiliser et d'appliquer la formulation avant que celle-ci ne devienne trop visqueuse, voire solide, correspond en général pour les systèmes liquides étudiés à un doublement de la viscosité. Ce temps est proche du temps nécessaire au développement du maximum d'exothermie, c'est pour cette raison que le temps d'atteinte de la température maximale lors de la réticulation a été comparé, pour une formulation résine époxy-durcisseur et pour cette même formulation additivée de la molécule porteuse de fonction associative selon l'invention.

Formulation A :
   - Résine époxy de base Epikote® 828 EL d'Hexion (résine sur base bis-phénol A) : 100 g
   - Durcisseur cetepox® 1312 NFH d'Aditya Birla Chemicals (mélange d'Isophorone diamine CAS N° 2855-13-2 et d'alcool benzylique, ce mélange étant donné pour plus réactif vis-à-vis des résines époxy que l'isophorone diamine pure) : 62,2g
Formulation B : Formulation A avec en plus 6,5 g de 1-(2-aminoéthyl)imidazolidine-2-one CAS N° 6281-42-1, soit 4% poids de la résine finale
Formulation C : Formulation A avec en plus 11,3 g de 1-(2-aminoéthyl)imidazolidine-2-one, soit 7% poids de la résine finale
Formulation D :
   - Résine époxy de base Epikote® 828 EL : 100 g
   - Durcisseur Epikure® 943 d'Hexion (Isophorone diamine pure) : 23,2g
Formulation E : Formulation D avec en plus 4,9g de 1-(2-aminoéthyl)imidazolidine-2-one, soit 4% poids de la résine finale
Formulation F : Formulation D avec en plus 8,6g de 1-(2-aminoéthyl)imidazolidine-2-one soit 6,5% poids de la résine finale
Formulation G :
   - Résine époxy de base Epikote® 240 d'Hexion (mélange de résine époxy sur base bis-phénol A et de résine époxy sur base bis-phénol F, en présence d'un diluant réactif constitué de molécules linéaires de fonctionnalité monoglycidyle en C10-C14) : 100g
   - Durcisseur Cetepox® 1312 NFH : 62,2g
Formulation H : Formulation G avec en plus 6,5g de 1-(2-aminoéthyl)imidazolidine-2-one, soit 4% poids de la résine finale

Les résultats des temps d'éxothermie maximale (en minutes) sont rassemblés dans le tableau 1 ci-dessous :

**Tableau 1**

| Formulation | A | B | C | D | E | F | G | H |
|---|---|---|---|---|---|---|---|---|
| Température maximale enregistrée en °C | 106°C | 106°C | 133°C | 133°C | 169°C | 172°C | 66°C | 104°C |
| Temps à la température maximale, en minutes | 62' | 40' | 30' | 167' | 117' | 58' | 96' | 34' |

On voit que l'adjonction de quelques pourcent de 1-(2-aminoéthyl)imidazolidine-2-one aux formulations liquides A, D ou G permet une réduction du temps d'atteinte de l'exothermie maximale qui va de 35% environ pour la formulation A (en comparaison avec C) et jusqu'à 65% pour D (en comparaison avec F) et G (en comparaison avec H), ce qui permet de compter sur un raccourcissement du temps ouvert de chaque formulation dans les mêmes proportions. On note que l'effet d'accélération de la réticulation par l'emploi de 1-(2-aminoéthyl)imidazolidine-2-one avec le durcisseur commercial est le plus important pour les formulations D et G qui sont les moins réactives des systèmes époxy testées.

### Exemple 2 (illustratif): Accélération de la prise d'une résine époxy : comparaison avec un catalyseur amine tertiaire

La comparaison a porté sur l'utilisation du catalyseur tétraméthylpropylènediamine (TMPDA) CAS N°110-95-2 qui possède une masse moléculaire très proche de celle du 1-(2-aminoéthyl)imidazolidine-2-one (130 g/mole contre 129 g/mole respectivement) et qui du fait de ses deux fonctions amine tertiaire est considéré comme un bon catalyseur pour résines époxy. La TMPDA utilisée est commercialisée par Arkema France sous son nom usuel de tétraméthylpropylènediamine.
Formulation G = Formulation G de l'exemple 1
Formulation I = Formulation G de l'exemple 1 avec en plus 4% en poids de TMPDA
Formulation J = Formulation G de l'exemple 1 avec en plus 4% poids de 1-(2-aminoéthyl)imidazolidine-2-one
Formulation K = Formulation D de l'exemple 1
Formulation L = Formulation D de l'exemple 1 avec en plus 4% poids de TMPDA
Formulation M = Formulation D de l'exemple 1 avec en plus 4% poids de 1-(2-aminoéthyl)imidazolidine-2-one, c'est à dire comme la formulation E de l'exemple 1
Les résultats des temps en minutes pour atteindre le maximum d'exothermie sont donnés dans le tableau 2 ci-dessous.

**Tableau 2**

| Formulation | G | I | J | K | L | M |
|---|---|---|---|---|---|---|
| Temps à la température maximale, en minutes | 96' | 60' | 34' | 167' | 140' | 117' |

L'emploi de 1-(2-aminoéthyl)imidazolidine-2-one permet de diminuer le temps de prise des formulations testées G et K de façon plus importante qu'avec le bi-catalyseur TMPDA de masse moléculaire similaire.

### Exemple 3 (illustratif) : Flexibilisation d'une résine époxy

La flexibilité d'une résine réticulée peut être mesurée de plusieurs façon. La mesure de sa dureté Shore a été effetuée selon la norme Norme NF T 51109.

Les mesures de dureté Shore ont été obtenues avec un duromètre à aiguille de la marque Andilog®. L'appareil est étalonné pour obtenir une dureté de 100 pour un acier trempé à 0,9 % de carbone et de 35 pour les aciers doux. Afin de vérifier que la flexibilité de la résine finale par utilisation de la molécule porteuse de la fonction associative selon l'invention se conservait dans le temps nous avons maintenu chaque résine au four à 50°C pendant plusieurs jours et mesuré sa dureté Shore régulièrement (Norme NF T 51109). Selon ce test, le maintien à 50°C pendant 10 jours correspond environ à 6 mois de vieillissement à température ambiante.
Formulation A = formulation A de l'exemple 1
Formulation B = formulation B de l'exemple 1
Formulation C = formulation C de l'exemple 1

Résultats exprimés en dureté Shore en fonction du temps à 50°C, en jours. Le temps zéro correspond à la mesure de dureté après réaction entre la résine de base et le durcisseur contenant la 1-(2-aminoéthyl)imidazolidine-2-one (formulations B et C) ou non (formulation A), c'est à dire après développement complet de l'exothermie causée par la réticulation, et retour à température ambiante de la résine. Les mesures portées dans le tableau 3 sont les moyennes de 5 mesures de dureté

**Tableau 3**

| Formulation | A | B | C |
|---|---|---|---|
| Dureté au temps 0 | 23,5 | 14 | 21 |
| Dureté à 3 jours à 50°C | 22,9 | 15,9 | 22,1 |
| Dureté à 4 jours à 50°C | 24,2 | 17 | 20,1 |
| Dureté à 10 jours à 50°C | 26,8 | 18,1 | 23,3 |

L'abaissement global de la dureté de la formulation B par rapport à la référence A de l'exemple 3 montre l'effet flexibilisant sur la résine finale de l'utilisation de 4% de la molécule porteuse de la fonction associative selon l'invention. Cet effet persiste au cours du test de vieillissement que constitue le maintien de la résine à chaud pendant 10 jours.

### Exemple 4 selon l'invention : Renforcement de l'adhérence d'une résine époxy.

L'amélioration de l'adhérence d'une résine époxy finie sur divers supports grâce à l'utilisation d'une molécule porteuse de fonction associative selon l'invention a été mesurée selon un test dit d'arrachage, Norme ISO 2409.

Le principe consiste à effectuer un quadrillage sur résine dont la reticulation a eu lieu sur différents supports, en réalisant des incisions parallèles et perpendiculaires dans la résine. Le quadrillage est constitué de 25 carrés de 1mm sur 1mm et de 100µm d'épaisseur. Les incisions doivent pénétrer jusqu'au support du film de peinture. On place une bande de ruban adhésif sur le quadrillage réalisé que l'on arrache rapidement après 5 minutes. On caractérise alors l'adhérence par le nombre de petits carreaux arrachés par le ruban. Moins il y a de carreaux arrachés, meilleure est jugée l'adhérence.
Formulation N :
   - Résine de base Epikote® 828 EL : 25g
   - Durcisseur Ancamine® 2609 (Eurochem Kimya) : 10g. Ancamine® 2609 est un mélange d'alkyl- et d'arylamines di-primaires, c'est à dire possédant deux functions -NH2 réactantes des époxydes, catalysé par un phénol (p-tBu phenol)
Formulation O = Formulation N incorporant en plus de la 1-(2-aminoéthyl)imidazolidine-2-one (1,45g , soit 4% poids de la résine finale)
Formulation P = Formulation N incorporant en plus 2,6 g de 1-(2-aminoéthyl)imidazolidine-2-one (7% poids de la résine finale)

Les résultats du test d'arrachement sur divers supports, en nombre de carrés restant adhérés après arrachage du ruban sont rassemblés dans le tableau 4 suivant.

**Tableau 4**

| Formulation | N | O | P |
|---|---|---|---|
| Nombre de carrés restant sur support céramique de surface très fermée | 1 | 15 | 18 |
| Nombre de carrés restant sur support Polyméthacrylate de méthyle (PMMA) | 1 | 5 | 15 |
| Nombre de carrés restant sur Acier carbone XC18 | 1 | 5 | 10 |

Les formulations O et P présentent un arrachage bien moindre que la référence N, l'incorporation dans la formulation initiale de quelques pourcents poids de 1-(2-aminoéthyl)imidazolidine-2-one améliore très nettement l'adhérence de la résine époxy finie sur céramique, acier et polymère présentant des groupements carbonyles tels que le PMMA.

## Revendications

1. Utilisation en tant que durcisseur de résine thermodurcissable, d'une molécule porteuse de groupe associatif de formule (I) : 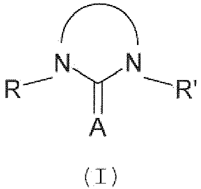 dans laquelle :
R désigne un motif contenant au moins une fonction réactive choisie parmi une fonction alcool ou amine,
R¹ désigne un atome d'hydrogène,
A désigne un atome d'oxygène,
en combinaison avec un co-durcisseur portant des fonctions amine ou alcool, pour augmenter l'adhérence à un support de la résine thermodurcissable.

2. Utilisation selon la revendication 1, **caractérisée en ce que** R est choisi parmi H₂N-(CH₂)ₙ- et HO-(CH₂)ₙ- où n représente un entier compris entre 1 et 18.

3. Utilisation selon la revendication 1 ou 2, **caractérisé en ce que** ladite molécule porteuse de groupe associatif est choisie parmi : la 1-(2-aminoéthyl)imidazolidone, la 1-(2-hydroxyéthyl)imidazolidone, 1-(2-[(2-aminoéthyl)amino]éthyl)imidazolidone, la 1-(2-{2-[(2-aminoéthylamino]éthyl}amino) éthyl]imidazolidone, la N-(6-aminohexyl)-N'-(6-méthyl-4-oxo-1,4-dihydropyrimidin-2-yl)urée.

4. Utilisation d'une molécule selon l'une quelconque des revendications 1 à 3, **caractérisée en ce que** le co-durcisseur est choisi parmi les alkyl- ou aryl- amines.

5. Utilisation d'une molécule selon l'une quelconque des revendications 1 à 4, **caractérisée en ce que** le co-durcisseur est choisi parmi l'éthylène diamine, la diéthylène triamine, la triéthylène tétramine et la tétraéthylène pentamine, les diamines cycliques notammment la 1,2 diamino cyclohexane, l'isophorone diamine, la N,N'-diisopropyle isophorone diamine, les diamines primaires aromatiques notamment la 4,4' méthylène dianiline, la méthylene bis 4,4' orthochloroaniline, les isomères de xylène diamine, telles que la diéthyltoluènediamine, l'hydroxyéthyldiéthylène triamine, les polyétheramines, les adduits DGEBA-amines aliphatiques en excès de fonctions amine relativement aux glycidyles, les polyamidoamines, les amidoamines, les bases de Mannich obtenus par réaction entre (poly)amine, formaldéhyde et (alkyl)phénols, les résines époxy polyols, et les polyols durcisseurs de polyuréthane.

6. Utilisation selon l'une quelconque des revendications 1 à 5, **caractérisée en ce que** la résine thermodurcissable est choisie parmi les résines époxy, les résines polyuréthanes, les résines polyester et polyester insaturés.

7. Utilisation selon l'une quelconque des revendications 1 à 6, dans laquelle aux moins deux résines thermodurcissables sont mises en oeuvre.

8. Utilisation selon la revendication 7, **caractérisée en ce que** les deux résines sont différentes.

9. Utilisation selon l'une quelconque des revendications précédentes, **caractérisée en ce que** les molécules porteuses de groupements associatifs sont utilisées en combinaison avec le co-durcisseur à hauteur de 0,1 à 50% en poids par rapport au poids total de la combinaison.

10. Utilisation selon l'une quelconque des revendications 2 à 9, **caractérisée en ce que** n est égal à 1 ou 2, et **en ce que** la résine thermodurcissable se trouve dans l'eau ou dans une émulsion aqueuse.

## Patentansprüche

1. Verwendung eines Moleküls mit einer assoziativen Gruppe der Formel (I): 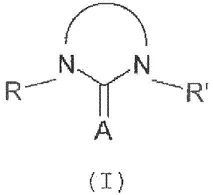 in der:
R für eine Einheit mit mindestens einer reaktiven Funktion, die aus einer Alkohol- oder Aminfunktion ausgewählt ist, steht,
R' für ein Wasserstoffatom steht,
A für ein Sauerstoffatom steht,
als Härter für ein wärmehärtbares Harz in Kombination mit einem Co-Härter mit Amin- oder Alkoholfunktionen zur Verbesserung der Haftung des wärmehärtbaren Harzes auf einem Träger.

2. Verwendung nach Anspruch 1, **dadurch gekennzeichnet, dass** R aus H₂N-(CH₂)ₙ- oder HO-(CH₂)ₙ-ausgewählt ist, wobei n für eine ganze Zahl zwischen 1 und 18 steht.

3. Verwendung nach Anspruch 1 oder 2, **dadurch gekennzeichnet, dass** das Molekül mit einer assoziativen Gruppe aus 1-(2-Aminoethyl)imidazolidon, 1-(2-Hydroxyethyl)imidazolidon, 1-(2-[(2-Aminoethyl)-amino]ethyl)imidazolidon, 1-(2-{2-[(2-Aminoethyl-amino]ethyl}amino)ethyl]imidazolidon und N-(6-Aminohexyl)-N'-(6-methyl-4-oxo-1,4-dihydro-pyrimidin-2-yl)harnstoff ausgewählt ist.

4. Verwendung eines Moleküls nach einem der Ansprüche 1 bis 3, **dadurch gekennzeichnet, dass** der Co-Härter aus Alkyl- oder Arylaminen ausgewählt ist.

5. Verwendung eines Moleküls nach einem der Ansprüche 1 bis 4, **dadurch gekennzeichnet, dass** der Co-Härter aus Ethylendiamin, Diethylentriamin, Triethylentetramin und Tetraethylenpentamin, cyclischem Diaminen, insbesondere 1,2-Diaminocyclohexan, Isophorondiamin, N,N'-Diisopropylisophorondiamin, aromatischen primären Diaminen, insbesondere 4,4'-Methylendianilin, Methylenbis-4,4'-orthochloranilin, Xyloldiamin-Isomeren, wie Diethyltoluoldiamin, Hydroxyethyldiethylentriamin, Polyetheraminen, Addukten von DGEBA und aliphatischen Aminen mit einem Überschuss an Amin-Funktionen gegenüber den Glycidylfunktionen, Polyamidoaminen, Amidoaminen, durch Reaktion zwischen (Poly)amin, Formaldehyd und (Alkyl)phenolen erhaltenen Mannich-Basen, Polyolepoxidharzen und polyurethanhärtenden Polyolen ausgewählt ist.

6. Verwendung nach einem der Ansprüche 1 bis 5, **dadurch gekennzeichnet, dass** das wärmehärtbare Harz aus Epoxidharzen, Polyurethanharzen, Polyesterharzen und ungesättigten Polyesterharzen ausgewählt ist.

7. Verwendung nach einem der Ansprüche 1 bis 6, wobei mindestens zwei wärmehärtbare Harze verwendet werden.

8. Verwendung nach Anspruch 7, **dadurch gekennzeichnet, dass** die beiden Harze verschieden sind.

9. Verwendung nach einem der vorhergehenden Ansprüche, **dadurch gekennzeichnet, dass** die Moleküle mit assoziativen Gruppen in Kombination mit dem Co-Härter in einem Gehalt von 0,1 bis 50 Gew.-%, bezogen auf das Gesamtgewicht der Kombination, verwendet werden.

10. Verwendung nach einem der Ansprüche 2 bis 9, **dadurch gekennzeichnet, dass** n gleich 1 oder 2 ist und das wärmehärtbare Harz in Wasser oder in wässriger Emulsion vorliegt.

## Claims

1. Use, as curing agent for a thermosetting resin, of a molecule carrying an associative group of formula (I) : 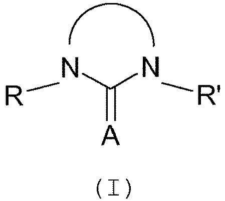 in which:
R denotes a unit comprising at least one reactive functional group chosen from an alcohol or amine functional group,
R' denotes a hydrogen atom,
A denotes an oxygen atom,
in combination with a cocuring agent carrying amine or alcohol functional groups, for improving the adhesion of the thermosetting resin to a support.

2. Use according to Claim 1, **characterized in that** R is chosen from H₂N-(CH₂)ₙ-, and HO-(CH₂)ₙ-, where n represents an integer between 1 and 18.

3. Use according to Claim 1 or 2, **characterized in that** said molecule carrying an associative group is chosen from: 1-(2-aminoethyl)imidazolidone, 1-(2-hydroxyethyl)imidazolidone,1-(2-[(2-aminoethyl)amino]ethyl) imidazolidone,1-(2-[2-{2-aminoethylamino}ethylamino]ethyl) imidazolidone or N-(6-aminohexyl)-N'-(6-methyl-4-oxo-1,4-dihydropy-rimidin-2-yl)urea.

4. Use of a molecule according to any one of Claims 1 to 3, **characterized in that** the cocuring agent is chosen from alkyl- or arylamines.

5. Use of a molecule according to any one of Claims 1 to 4, **characterized in that** the cocuring agent is chosen from ethylenediamine, diethylenetriamine, triethylenetetramine and tetraethylenepentamine, cyclic diamines, in particular 1-2-diaminocyclohexane, isophoronediamine or N,N'-diisopropylisophoronediamine,
aromatic primary diamines, in particular 4,4'-methylenedianiline, 4,4'-methylenebis(orthochloroaniline) or xylenediamine isomers, such as diethyltoluenediamine, hydroxyethyldiethylenetriamine, polyetheramines, BADGE-aliphatic amine adducts with an excess of amine functional groups relative to the glycidyls, polyamidoamines, amidoamines, the Mannich bases obtained by reaction between (poly)amine, formaldehyde and (alkyl)phenols, polyol epoxy resins and polyurethane-curing polyols.

6. Use according to any one of Claims 1 to 5, **characterized in that** the thermosetting resin is chosen from epoxy resins, polyurethane resins, polyester resins and unsaturated polyester resins.

7. Use according to any one of Claims 1 to 6, in which at least two thermosetting resins are employed.

8. Use according to Claim 7, **characterized in that** the two resins are different.

9. Use according to any one of the preceding claims **characterized in that** the molecules carrying associative groups are used in combination with the cocuring agent at a content of 0.1 to 50% by weight, with respect to the total weight of the combination.

10. Use according to any one of Claims 2 to 9, **characterized in that** n is equal to 1 or 2, and **in that** the thermosetting resin is present in water or in an aqueous emulsion.
